Europäisches Patentamt

European Patent Office

Office européen des brevets

(11) Publication number: 0 643 954 A1

# EUROPEAN PATENT APPLICATION

(21) Application number: 93307348.8

(51) Int. Cl.6: **A61F 13/02**

(22) Date of filing: **17.09.93**

(43) Date of publication of application:
**22.03.95 Bulletin 95/12**

(84) Designated Contracting States:
**DE FR GB**

(71) Applicant: **Tanaka, Nobutaka**
**8-9, Hojo 7-chome**
**Daito-shi,**
**Osaka-fu (JP)**

(72) Inventor: **Tanaka, Nobutaka**
**8-9, Hojo 7-chome**
**Daito-shi,**
**Osaka-fu (JP)**

(74) Representative: **Smith, Norman Ian et al**
**F.J. CLEVELAND & COMPANY**
**40-43 Chancery Lane**
**London WC2A 1JO (GB)**

(54) **Adhesive plaster.**

(57) A plaster which is applied to an afflicted part of the human body in order to abate a muscular ache or swelling. The plaster is made of a mesh-like sheet (1) having an adhesive face and perforated with many uniform square or rectangle or lozenge holes (10). One side of the hole measures in the range of from 3 millimeters to 7 millimeters and an interval between the facing sides of two holes measures in the range of from 2.5 millimeters to 3 millimeters.

FIG. 5

This invention relates to a plaster which is applied to a trouble part of the human body in order to relieve muscular ache or swelling .

A taping therapy, which means that plasters are applied to trouble parts on the human body, is well known as one of the method of medical treatment for a sprain or a bruise. This medical treatment is operated at an oriental osteopathy clinic where an oriental medical therapy such as an acupuncture, a cauterization with moxa,and a finger-pressure treatment are in effect .

A curative value by the taping treatment can be proved by means of a gate control theory, an axis sylinder reflex theory,a somatic autonomic reflex theory,and the others. Concretely they say that a fascia protective action by means of the taping therapy enhances the curative value.

Generally, a strain of muscle is often caused by a lack of exercise,an external shock,senility,stress and so on.And then a muscular ache is caused by the strain of muscles. In order to endure the pain,the strain of the muscles is invited again, and the condition of the disease develops into a myalgia by the muscular rigor and develops further into a cellulitis .

An usual plaster used for the taping treatment in order to abate the muscular ache or swelling is a simple non-pored sheet having an adhesive face.

Otherwise,the Oriental medical science says that there are a large number of important points on the human body ,which are connected with the internal organs or tissues and control their functions . These points are called "tsubo" in Japan.

The non-pored sheet is applied to the disease parts or the "tsubo" related to the illness on the human body. Thereby the pain of the muscles can be relieved .

Above mentioned usual plaster,however,is not sufficient for the remedial value.

The problem is as follows;

The former non-pored plaster have a function to protect the malady parts or the "tsubo" related to the disease on the human body. Thereby,a burden on the trouble parts or the "tsubo" can be relieved,and then a healing phenomenon by a natural healing force can proceed smoothly in the human body .

However,since the non-pored plaster is no more than a protective sheet which protect the disease parts on the human body until natural recovery from disease , it is difficult to expect a positive and early recovery. Accordingly the remedial value by the former non-pored plaster is still inadequate.

In view of the foregoing,an object of the invention is to provide a plaster which is applied to a disease part involving a pain or a swelling or to a "tsubo" related to the illness on the human body and which can hasten the recovery of the ailment.

To achieve this object ,the plaster of this invention is made of a mesh-like sheet having an adhesive face.

The function of the plaster will be clarified in detail.

The plaster made of the mesh-like sheet is applied to the disease part on the human body. Thus, since the disease part is covered by the mesh-like plaster, the disease part on the human body is protected by it as same as by the above mentioned usual non-pored plaster. However,when the applicated condition of the plaster in this invention to the human body is observed in detail,there is a distinction between the mesh-like sheet and the non-pored sheet. An adhesion of the mesh-like sheet is concentrated under lines around meshes and under the cross-points of the lines. Namely,the adhesion of the plaster acts to the trouble part on the human body partially,in other words,the adhesion of the mesh-like plaster operates to the disease part on the human body as a dynamical burden.

The trouble is caused by the very strong strain or relaxation of the muscles, and when the mesh-like plaster is applied to the trouble part on the human body,the musclar strain by the adhesive parts of the plaster and the musclar relaxation by the un-adhesive parts of the plaster produce good strained condition to the muscles. Namely,a formation of a sodium element in the disease part can be promoted by the dynamical burden and a satiration period of the sodium element is advanced. Accordingly a neutralization starting from the satiration period of the sodium element i.e. the natural healing fenomenon can start and advance in an early stage, and the pain of the muscles abates in the early time.

A consequence of a comparison test which compares the remedial value by the mesh-like plaster in this invention with by the former non-pored plaster will explain as shown in a table 1.

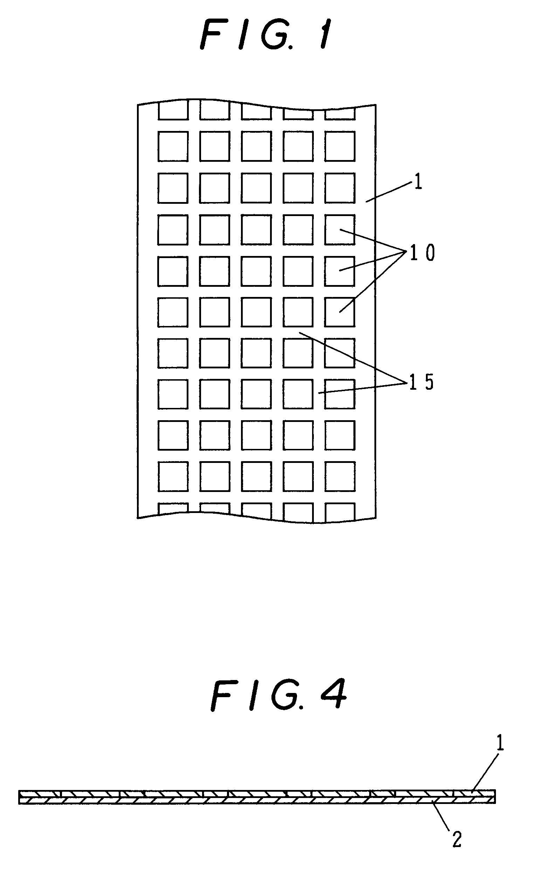

A plaster used in the comparison test is perforated with many square holes uniformly as shown in Fig.1.The plaster is 57 millimeters in width and one side of the square hole is 7 millimeters long. And the square holes are arranged at 3-millimeter intervals lengthwise and sidewise.

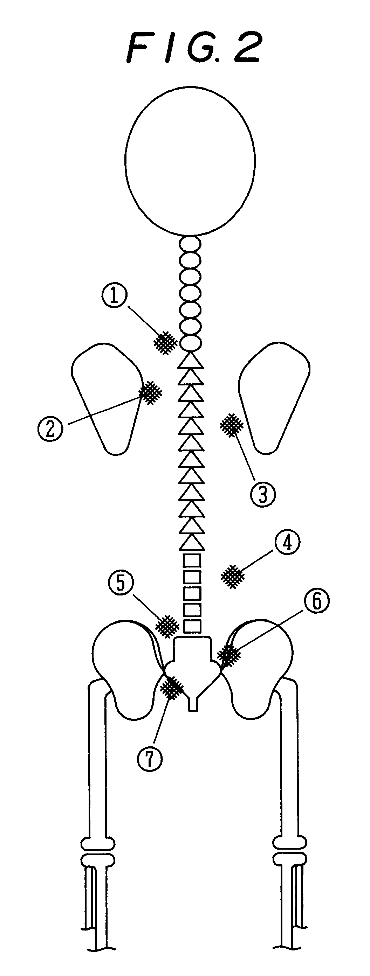

The comparison test was operated to two patients who complained of pain in their neck and waist.The healing degree of ailments in the neck and the waist can be verified by a measurement of a grasping power. The plasters were applied to the following seven points of the person's body as shown in Fig.2.

The first point ① indicates an application place remote from the seventh cervical vertebra to the left by two fingers width .

The second point ② indicates an application place remote from the third thoracic vertebrae to the right by four and a half fingers width .

The third point ③ indicates an application place remote from the fifth thoracic vertebrae to the right by four and a half fingers width .

The fourth point ④ indicates an application place remote from the second lumbar vertebrae to the right by four and a half fingers width .

The fifth point ⑤ indicates an application place remote from the fifth lumbar vertebrae to the left by two fingers width .

The sixth point ⑥ indicates the upper right of the epigaster.

The seventh point ⑦ indicates the lower left outside of the sacrum.

One patient is a male and 49 years old and the other patient is a female and 39 years old. The test was operated at thirty-second intervals.

As shown in table 1,considering from the measured values of the male patient,when he was applying the non-pored plasters to his body,the measured value of the grasping power of his left arm was 48.0 kg.In comparison with that value,when he applied the mesh-like plasters instead of the non-pored plasters to the designated same places on his body,the measured value of the grasping power of his left arm has improved to 49.5 kg. ~ 52.5 kg.

| step | measured values of graping power | |
| --- | --- | --- |
| | patient (male) | patient (female) |
| before application | left arm :43.5 kg.<br>right arm:43.0 kg. | left arm :27.0 kg.<br>right arm:29.5 kg. |
| under application of non-pored plasters | left arm :48.0 kg.<br>right arm:48.5 kg. | left arm :31.0 kg.<br>right arm:34.0 kg. |
| under application of mesh-like plasters (at the first time) | left arm :49.5 kg.<br>right arm:49.5 kg. | left arm :31.0 kg.<br>right arm:36.0 kg. |
| ditto (at the second time) | left arm :52.0 kg.<br>right arm:51.0 kg. | left arm :31.0 kg.<br>right arm:36.0 kg. |
| ditto (at the third time) | left arm :52.5 kg.<br>right arm:54.0 kg. | left arm : ／<br>right arm: ／ |

(table 1 )

That is to say,even if the burden for his neck and waist became heavy,his muscles in said portions were able to withstand the heavy burden. This means that the trouble in his neck and waist has recovered. And in the case of his right arm,too,the measured value of the grasping power under application the former plaster is 48.5 kg.,and after application the plaster in this invention, the values were raised up to 49.5 kg. ~ 54.0 kg.

Besides,As for the female patient,too,it is obvious that the same effectiveness by the plaster in this invention as the male patient can be obtained

The effect of the invention is as follows;

As comparison with the non-pored plaster,the mesh-like plaster in this invention can cure a trouble in a muscles of the human body speedily and efficaciously .

The invention will be more fully understood from the following description given by way of example only with reference to the several figures of the accompanying drawings in which,

Fig.1 is a plan view of a plaster as an embodiment of this invention.

Fig.2 is a schematic illustration of the human body designating application places of the plaster in acomparison test.

Fig.3 is a schematical view of a process manufacturing a plaster.

Fig.4 is a sectional view of a plaster of Fig.1.

Fig.5 is a schematical view of a mesh-like plaster separated from a separate paper.

Fig.6 is plan views of the other plasters.

[ EMBODIMENTS ]

Preferred embodiments of this invention are explained by the figures.

As shown in Fig.1 and Fig.4,a plaster in this invention is made of a mesh-like adhesive main sheet 1.In this embodiment, the main sheet 1 is 57 millimeters in width and perforated with many uniform square holes 10 arranged side by side. In other words,the main sheet 1 is a lattice-type sheet. The one side of the square hole 10 is 7 millimeters long.And the square holes 10 are arranged at 3-millimeter intervals. An adhesive face of the main sheet 1 is coverd by a separate paper 2.

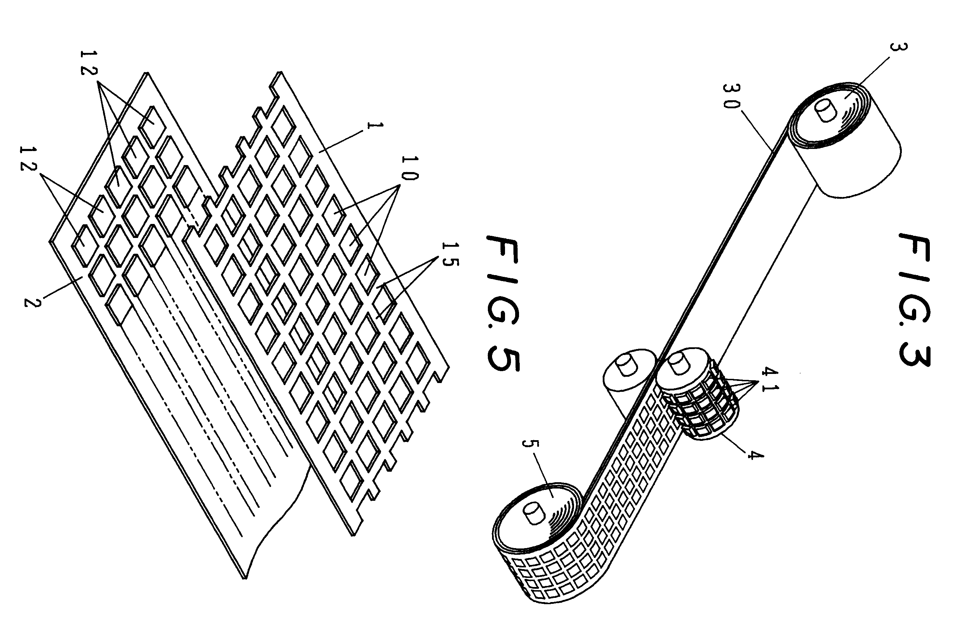

Next , a process of manifacturing of the mesh-like plaster will be explained with Fig.3 refering.

An original sheet 30 consists of the main sheet 1 having the adhesive face and the separate paper 2 covering the adhesive face of the sheet 1. The original sheet 30 is rolled up. And the roll of the original sheet 30 is called an original roll 3 .

The original sheet 30 is pulled out of the original roll 3 so that the main sheet 1 is on the separate paper 2. The punching roll 4 having many square cutting blades 41 is provided at a downstream of the original roll 3 which the original sheet 30 is pulled out .By means of the cutting blades 41,many square slittings which correspond to outlines of the square holes 10 are cut into only the main sheet 1 which is the upside of the original sheet 30. And after the punching,the original sheet 30 is rolled up again.Thus,a finished roll 5 is perfected.

When you need this plaster,you may pull the main sheet 1 having the square slittings and the separate paper 2 out of the finished roll 5 and may cut the sheet 1 in the necessary length. And when the separate paper 2 is separated from the adhesive face of the sheet 1 ,as shown in Fig.5, square scraps 12 which correspond to the square holes 10 are left over on the separate paper 2. Thus, the main sheet 1 is perforated with the square holes 10.

The length of the one side of the square hole 10 is not restricted to 7 millimeters. It is allowed in the range of from 3 millimeters to 7 millimeters. If the sizes of the hole 10 are within the above mentioned ranges,the remedial values by these plasters can be gained on the same level.

And the interval 15 between two square holes measures in the range of from 2.5 millimeters to 3 millimeters in width. And then,the widths of the interval 15 between two holes and the lengths of the one side of the hole 10 may be combined suitably within the above mentioned extent.

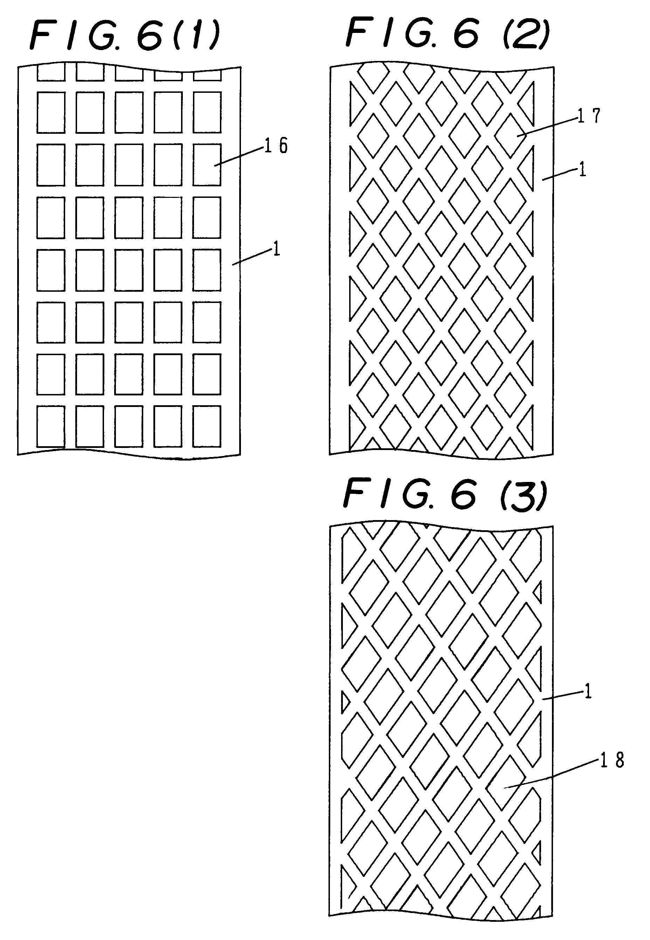

Besides,the shape of the hole perforated uniformly in the main sheet 1 is not restricted to square,any shape will do, for example, rectangle holes 16 as shown in Fig.6-(1), lozenge holes 17 as shown in Fig.6-(2), and parallelogram holes 18 as shown in Fig.6-(3),provided that the one side of the each hole measures in the range of from 3 to 7 millimeters, and that the interval between facing sides of the two holes measures in the ranges of from 2.5 to 3 millimeters.

## Claims

1. A plaster for application to a troubled or damaged part of the human body in order to abate a muscular ache or swelling, characterised in that the plaster is made of a mesh-like sheet having an adhesive face and being perforated with a plurality of generally uniform holes.

2. A plaster as claimed in claim 1, wherein the mesh-like sheet is in the form of a punched sheet.

3. A plaster as claimed in claim 1, wherein teh plaster is perforated with uniform square holes.

4. A plaster as claimed in claim 1, wherein the plaster is perforated with uniform rectangular holes.

5. A plaster as claimed in claim 1, wherein the plaster is perforated with uniform lozenge-type holes.

6. A plaster as claimed in any one of claims 3 to 5, wherein one side of each hole measures in the range of from three to seven millimeters, and an interval between facing sides of the two adjacent holes measures in the range of from two point five to three millimeters.

7. A plaster as claimed in claim 3 or claim 5, wherein one side of each hole is seven millimeters long and facing sides of the two holes are arranged at three-millimeter intervals.

# F I G. 1

# F I G. 4

# F I G. 2

# FIG. 3

# FIG. 5

# F I G. 6 (1)

16

1

# F I G. 6 (2)

17

1

# F I G. 6 (3)

1

18

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | US-A-2 096 564 (W.M.SCHOLL) <br> * page 1, left column, line 35 - right column, line 7; figures 3-4 * <br> --- | 1-7 | A61F13/02 |
| X | US-A-2 807 262 (R.B.LEW) <br> * column 1, line 35 - line 40; figures * <br> --- | 1-7 | |
| X | US-A-3 214 502 (C.H.SCHAAR) <br> * column 3, line 13 - line 15; figures 1-2,8 * <br> --- | 1-7 | |
| X | DE-A-25 17 790 (LOHMANN) <br> * claims 1,2; figure * <br> --- | 1-7 | |
| X | CH-A-332 999 (LOHMANN) <br> * claims 1,2; figures * <br> ----- | 1,2 | |
| | | | TECHNICAL FIELDS SEARCHED (Int.Cl.6) |
| | | | A61F |

The present search report has been drawn up for all claims

| Place of search | Date of completion of the search | Examiner |
|---|---|---|
| THE HAGUE | 19 January 1994 | Nice, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document